# FASCICULE DE BREVET EUROPEEN

(11) **EP 2 421 884 B1**
(45) Date de publication et mention de la délivrance du brevet: **18.03.2015**
(21) Numéro de dépôt: 10718242.0
(22) Date de dépôt: 22.04.2010
(51) Int. Cl.: C07K 5/08, C07K 5/10, C07K 7/06, C07K 14/395, C07K 14/415, A61K 8/64, A61Q 19/08, C07K 5/093, C07K 5/103, C07K 5/113

(54) **HYDROLYSATS PEPTIDIQUES ACTIVATEURS DU PROTEASOME ET COMPOSITIONS LES CONTENANT**
PEPTIDHYDROLYSAT ALS PROTEASOMAKTIVATOR UND ZUSAMMENSETZUNGEN, DIE DIESES ENTHALTEN
PEPTIDE HYDROLYSATE PROTEASOME ACTIVATORS AND COMPOSITIONS CONTAINING SAME

(30) Priorité: 23.04.2009 FR 0901978
(43) Date de publication de la demande: 29.02.2012
(73) Titulaire: ISP Investments Inc., Wilmington, DE 19805 (US)
(72) Inventeur: DAL FARRA, Claude, Kerhonskon NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000324
(87) Numéro de publication internationale: WO 2010/122244

(56) Documents cités:
- WO-A2-2008/015343
- FR-A1- 2 822 701
- FR-A1- 2 904 552
- FR-A1- 2 915 378
- FR-A1- 2 915 379
- FR-A1- 2 915 380
- FR-A1- 2 915 381
- FR-A1- 2 915 382
- FR-A1- 2 915 383
- FR-A1- 2 915 384

## Description

La présente invention se rapporte au domaine des actifs anti-âge et de leurs utilisations en cosmétique notamment. Plus particulièrement la présente invention se rapporte à un hydrolysat peptidique enrichi en peptides bioactifs, ledit hydrolysat étant activateur du proteasome, ainsi que son utilisation en cosmétique et/ou pharmaceutique afin de prévenir et/ou corriger les effets du vieillissement et du photo-vieillissement de la peau et des phanères, et protéger la peau contre les agressions dues aux rayonnements UV.

Le vieillissement correspond à l'ensemble des processus physiologiques et psychologiques qui modifient la structure et les fonctions de l'organisme à partir d'un certain âge. On distingue deux types de vieillissement, d'une part, le vieillissement intrinsèque et, d'autre part, le vieillissement extrinsèque. Le vieillissement intrinsèque est dû à des facteurs génétiques, à des modifications biochimiques qui ont lieu lors d'états de fatigue, de stress, de changements hormonaux comme lors de la grossesse... Le vieillissement extrinsèque, quant à lui, est dû à des facteurs environnementaux auxquels est soumis l'organisme tout au long de sa vie, tels que la pollution, le soleil, les maladies etc. Il s'agit d'un processus lent et progressif qui atteint toutes les cellules de l'organisme par différents moyens et se manifeste de différentes manières. Par exemple, au niveau de la peau, l'aspect de celle-ci est modifié par les divers types d'agressions internes ou externes et l'on voit apparaître alors des rides et ridules, des taches d'hyper ou d'hypo-pigmentation, une sécheresse voire une déshydratation de la peau, un amincissement de l'épiderme, une élastose, des imperfections, des taches de vieillesse.... Tous ces changements affectent non seulement la peau, mais également les phanères tels que les ongles et les cheveux. Ces modifications sont dues entre autres à l'altération des fonctions de renouvellement cellulaire, de cohésion cellulaire, de synthèse de collagène, d'élastine et d'autres protéines, et conduisent finalement à une diminution des qualités de barrière protectrice de la peau et à un aspect peu esthétique de celle-ci. Mais l'un des processus principaux responsables du vieillissement des cellules est sans conteste l'accumulation de protéines endommagées dans ces dernières. En effet, les protéines sont la cible de diverses modifications post-traductionnelles anormales telles que l'oxydation, la glycation, la conjugaison avec des produits issus de la peroxydation lipidique, phénomènes dont l'incidence augmente fortement avec l'âge.

Il est connu que les radicaux libres jouent un rôle clé dans le processus de vieillissement et plus particulièrement dans la formation de protéines oxydées, endommagées (Harman et al. « Aging: a theory based on free radical and radiation chemistry » J. Gerontol., 11, 298-300). L'accumulation de protéines endommagées pose donc le problème de l'efficacité des systèmes protéolytiques en charge de l'élimination de ces protéines, et particulièrement celle du système protéosomal impliqué non seulement dans l'élimination des protéines altérées, notamment par oxydation, mais aussi dans le renouvellement continu des protéines intracellulaires.

La voie ubiquitine-protéasome joue un rôle fondamental dans un très grand nombre de processus biologiques. En effet, les mécanismes de dégradation des protéines par le protéasome sont impliqués dans des mécanismes cellulaires importants tels que la réparation de l'ADN, le contrôle de l'expression des gènes, la régulation de la progression du cycle cellulaire, le contrôle de la qualité des protéines néo-synthétisées, l'apoptose ou la réponse immunitaire (Glickman and Ciechanover, 2002).

Le protéasome présent dans les cellules humaines est un complexe multi protéique de très grande taille présent dans le cytoplasme et le noyau. Les formes purifiées du protéasome comprennent 2 grandes sous-unités ; d'une part, un coeur protéolytique appelé protéasome 2OS et, d'autre part, un complexe régulateur 19S qui se lie à chacune des deux extrémités du protéasome 2OS (Coux et *al.,* 1996 ; Glickman et Coux, 2001). Le protéasome 2OS est une particule en forme de cylindre creux, composée de 28 sous-unités alpha et béta, distribuées en 4 anneaux heptamériqués. Les activités peptidases sont présentes à la surface interne du cylindre et s'influencent de manière allostérique. Trois activités protéolytiques (« trypsine, chymotrypsine et caspase-like ») ont été associées au protéasome 2OS et concourent à la destruction des protéines en peptides inactifs de 3 à 20 acides aminés. Outre le protéasome 2OS le protéasome 26S comprend le complexe régulateur 19S de 0,7 MDa constitué de 20 sous-unités environ. Des études récentes d'immunopurification ont montré que d'autres protéines pouvaient être associées au protéasome 20S et 19S (par exemple le complexe régulateur 11S.).

Des travaux menés ces dernières années ont permis de corréler le vieillissement avec l'activité du protéasome. En effet, alors qu'avec l'âge il y a une augmentation de l'accumulation des protéines oxydées, on a constaté une baisse de l'efficacité du système protéosomal (Petropoulos et al., J. Gerontol. A. Biol. Sci. 2000, 55A :B220-7). Cette baisse de l'efficacité du système protéosomal était due en fait à une baisse de la quantité de protéasome. Ces résultats ont été confirmés par ceux d'une étude portant sur la quantité et l'activité du protéasome dans des cellules d'individus centenaires versus individus jeunes (Chondrogianni et al., Exp. Gerontol. 2000 ;35 :721-8). Ces études, ainsi que bien d'autres, démontrent bien le lien entre vieillissement et activité du protéasome et l'on peut penser que l'induction de l'expression du protéasome dans des cellules de la peau ou des phanères pourrait avoir une influence positive sur le vieillissement, voire retarder celui-ci.

Dans l'optique de prévenir ou retarder le vieillissement, des compositions cosmétiques à base d'extraits naturels ont été proposées : par exemple le brevet FR 2822701 divulgue une composition à base d'un extrait d'algue phaeodactylum pour favoriser l'activité du protéasome. Ou encore, la demande de brevet FR 2898808 décrit l'utilisation d'une composition comprenant un extrait de micro-algue et de ferrulate d'arginine, toujours pour activer le protéasome. D'autres compositions quant à elle comprennent des composés chimiques, capables de moduler l'activité du protéasome pour avoir un effet antivieillissement. Ces compositions sont décrites dans les demandes de brevets WO 2006/105811 ou encore WO 2005/061530. Cependant les composés de type peptidique proposés présentent une taille importante et il est difficile dans le domaine de la cosmétique de les utiliser tels quels. Il existe alors un besoin, notamment dans l'industrie cosmétique, de nouvelles compositions provenant d'extraits naturels avec des principes actifs de taille réduite, et efficaces quant à leur utilisation en tant qu'activàteur du protéasome ayant un effet anti-âge.

C'est ainsi que la Demanderesse a découvert qu'un hydrolysat peptidique enrichi en peptides bioactifs était capable d'activer le protéasome et pouvait ainsi être utile afin de prévenir et/ ou traiter les signes cutanés du vieillissement et au photo-vieillissement, ainsi que les agressions dues aux rayonnements UV.

Par conséquent la présente invention a pour premier objet un hydrolysat peptidique activateur du protéasome, issu de l'hydrolyse de levures du genre *Saccharomyces,* et plus particulièrement de l'espèce *Saccharomyces cerevisiae,* ou de pois *(Pisum sativum)* et est enrichi en peptides bioactifs de poids moléculaire inférieur à 6 kDa, comportant de 3 à 5 acides aminés, chaque peptide bioactif comprenant au moins un résidu acide aspartique, un résidu cystéine et un résidu arginine.

La présente invention a pour second objet une composition cosmétique comprenant à titre de principe actif ledit hydrolysat peptidique enrichi.

En outre, la présente invention a pour troisième objet l'utilisation d'une composition cosmétique comprenant ledit hydrolysat peptidique enrichi pour prévenir et/ou traiter les signes cutanés du vieillissement et du photo-vieillissement et améliorer la dégradation par le protéasome des protéines endommagées.

Enfin, la présente invention a pour quatrième objet un procédé de traitement cosmétique de la peau ou des phanères à traiter à l'aide de la composition comprenant ledit hydrolysat peptidique enrichi.
La figure 1 représente un exemple de chromatogramme obtenu par CLHP, avec mise en évidence du pic correspondant au peptide bioactif dans un hydrolysat de maïs.
La figure 2 représente un exemple de chromatogramme obtenu par CLHP, avec mise en évidence du pic correspondant au peptide bioactif dans un hydrolysat de pois.
La figure 3 représente un exemple de chromatogramme obtenu par CLHP, avec mise en évidence du pic correspondant au peptide bioactif dans un hydrolysat de riz.
La figure 4 représente un exemple de chromatogramme obtenu par CLHP, avec mise en évidence du pic correspondant au peptide bioactif dans un hydrolysat de

### Saccharomyces cerevisiae

La demande de brevet WO 2008/015343 décrit des hydrolysats de levures pour augmenter la synthèse de mélanine dans les mélanocytes.

La présente invention a pour objet premier un hydrolysat peptidique activateur du protéasome, issu de l'hydrolyse de levures du genre *Saccharomyces,* et plus particulièrement de l'espèce *Saccharomyces cerevisiae,* ou de pois (*Pisum sativum*) et est enrichi en peptides bioactifs de poids moléculaire inférieur à 6 kDa, comportant de 3 à 5 acides aminés, chaque peptide bioactif comprenant au moins un résidu acide aspartique, un résidu cystéine et un résidu arginine.

On entend par « hydrolysat peptidique », un mélange de composés majoritairement représentés par des peptides ou des oligopeptides.

Par « peptides bioactifs » on entend un fragment de protéine composé par l'enchaînement d'au moins 3 acides aminés liés entre eux par des liaisons peptidiques modifiées ou non et qui présentent une activité en tant qu'activateur du protéasome. Présents sous forme inactive dans les protéines, ils deviennent actifs après hydrolyse de ces dernières.

Ledit hydrolysat peptidique enrichi selon l'invention est caractérisé en ce qu'il est activateur du protéasome.

On entend par hydrolysat peptidique (et/ou peptides bioactifs) « activateur du protéasome», tout hydrolysat peptidique, ou peptide ou dérivé biologiquement actif capable d'augmenter l'activité du protéasome, soit par augmentation de la synthèse protéique des sous-unités du protéasome (par modulation directe ou indirecte de l'expression génique), soit par d'autres processus biologiques tels que la stabilisation des sous-unités constituant le protéasome ou encore la stabilisation des transcrits d'ARN messager.

L'hydrolysat peptidique enrichi selon l'invention est caractérisé en ce qu'il permet d'activer la dégradation par le protéasome des protéines endommagées. Par « protéines endommagées » on entend des protéines ayant subi des réactions d'oxydation dues aux espèces réactives de l'oxygène (radicaux libres), des protéines glyquées ou conjuguées avec des produits issus de la péroxydation lipidique etc.

Préférentiellement, l'hydrolysat peptidique est enrichi en peptides bioactifs de formule générale (I) :

X₁ - [Asp, Cys, Arg] - X₂

Dans laquelle,
X₁ est une asparagine, une lysine, un aspartate, une valine, une arginine, ou est absent
X₂ est une histidine, une lysine, une arginine, ou est absent

Préférentiellement, l'hydrolysat peptidique est riche en peptides bioactifs de formule suivante :
(SEQ ID n°1) Arg - Asp -Cys -Arg - Arg
(SEQ ID n°2) Asn - Asp -Cys -Arg - Lys
(SEQ ID n°3) Asp -Cys -Arg - His
(SEQ ID n°4) Val - Asp -Cys -Arg
(SEQ ID n°5) Asp -Cys -Arg

En effet, ces peptides ont été identifiés comme étant particulièrement actifs en tant qu'activateurs du protéasome et présentent donc un intérêt particulier en tant qu'agent anti-âge.

L'hydrolysat peptidique enrichi selon l'invention est issu de l'hydrolyse de levures du genre Saccharomyces, et de manière préférentielle des levures de l'espèce *Saccharomyces cerevisiae*, ou de pois (*Pisum sativum*).

L'invention peut également être réalisée en utilisant l'une des nombreuses plantes de la famille des pois (Fabacées). On utilisera, par exemple, les plantes de l'espèce des pois *Pisum sativum L.* Le terme pois désigne en outre la graine, elle-même riche en protéines (25 %).

L'hydrolysat peptidique enrichi peut être obtenu à partir de diverses sources de protéines, qu'elles soient d'origine animale ou végétale. Selon un autre mode de réalisation, l'hydrolysat peptidique enrichi est issu de l'hydrolyse de plantes choisies parmi le maïs (*Zea mayz L*.), ou le riz (*Oryza sativa L*.). De préférence, les plantes utilisées ne sont pas soumises à une fermentation préalable.

Ainsi, on peut utiliser les graines d'une des nombreuses plantes du genre Zea et préférentiellement l'espèce *Zea mays L*. Le matériel végétal utilisé sera le grain et préférentiellement le grain débarrassé de son enveloppe par une étape de décorticage.

Les plantes de la famille du riz (Poacées), notamment celles du genre Oryza et plus préférentiellement l'espèce *Oryza sativa L* peuvent être utilisées pour réaliser l'hydrolysat. - Le matériel végétal utilisé sera le grain et préférentiellement le grain débarrassé de son enveloppe par une étape de décorticage.

Toute méthode d'extraction ou de purification connue de l'homme du métier peut être utilisée afin de préparer l'hydrolysat selon l'invention.

Dans une première étape, les graines, ou une partie spécifique de la plante (feuilles, tubercules, racines, etc.) sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

Lorsqu'il s'agit de levures, dans une première.étape, celles-ci sont mises en culture de manière classique dans un milieu adapté à leur développement, de préférence en présence de lactose. Elles sont récoltées par centrifugation puis mises en suspension dans une solution tampon, préférentiellement un tampon phosphate. Dans une seconde étape, ces cellules sont éclatées à l'aide d'une presse de French ou à l'aide d'un broyeur à bille, la majorité des composants membranaires insolubles étant écartés par centrifugation ou par filtration.

On réalise ensuite l'extraction des protéines suivant le procédé classique (Osborne, 1924) modifié ; le broyat de plante ou le lysat de levures est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromélaïne, ficine) et de micro-organismes (Aspergillus, Rhizopus, Bacillus, etc.). Les conditions d'hydrolyses sont choisies pour favoriser l'enrichissement en peptides bioactifs.

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après filtration, permettant d'éliminer les enzymes et les polymères, un premier filtrat est obtenu.

L'hydrolysat obtenu à ce stade peut être encore purifié afin de sélectionner les fractions de bas poids moléculaires, préférentiellement inférieures à 6 kDa, ainsi que les peptides générés selon leur nature. La purification s'effectue avantageusement par des étapes d'ultrafiltrations successives à travers des filtres de porosité décroissante, en conservant les filtrats à chaque étape et/ ou par une méthode de type chromatographique, afin d'enrichir spécifiquement l'hydrolysat en peptides bioactifs.

On procède à une phase de dilution dans de l'eau ou dans tout mélange contenant de l'eau, puis stérilisation par ultrafiltration afin d'obtenir un hydrolysat peptidique enrichi caractérisé par une teneur en protéines de 0,5 à 5,5 g/l. Cet hydrolysat peptidique enrichi correspond à la forme la plus purifiée du principe actif selon l'invention.

L'hydrolysat peptidique obtenu selon l'invention est analysé qualitativement et quantitativement en chromatographie liquide haute pression (CLHP), qui permet d'analyser les protéines ayant des poids moléculaires de 0,2 à 25 kDa (selon un gradient de solvants approprié). Les différentes fractions peptidiques qui ont pu être isolées sont ensuite analysées pour leur efficacité biologique. Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs.

Finalement, l'hydrolysat peptidique enrichi obtenu est composé de peptides de poids moléculaire inférieur à 6 kDa, et est enrichi en peptides bioactifs de 3 à 5 acides aminés comprenant au moins un résidu acide aspartique, un résidu cystéine et un résidu arginine.

Cet hydrolysat peptidique, selon l'invention, enrichi en peptides bioactifs peut en outre être utilisé à titre de médicament.

Le second objet de la présente invention se rapporte à une composition cosmétique comprenant à titre de principe actif l'hydrolysat peptidique enrichi en peptides bioactifs tels que décrits précédemment.

De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit hydrolysat peptidique est présent dans la composition en une quantité représentant de 0,0001 % à 20 % du poids total de la composition, et préférentiellement en une quantité représentant de 0,05 % à 5 % du poids total de la composition.

Dans les compositions selon l'invention, l'hydrolysat peptidique enrichi en peptides bioactifs est solubilisé dans un ou plusieurs solvants comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, l'hydrolysat peptidique selon l'invention est solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydroalcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotion, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition selon l'invention contient en outre, au moins un autre principe actif favorisant l'action dudit hydrolysat peptidique enrichi. On peut citer, de manière non limitative, les classes d'ingrédients suivantes : des agents actifs peptidiques, d'autres extraits de végétaux, des agents cicatrisants, anti-âge, anti-rides, apaisants, anti-radicalaires, anti-UV, des agents stimulant la synthèse de macromolécules dermiques ou le métabolisme énergétique, des agents hydratants, anti-bactériens, anti-fongiques, anti-inflammatoires, anesthésiques, des agents modulants la différenciation, la pigmentation ou la dépigmentation cutanée, des agents stimulants la pousse des ongles ou des cheveux.... Préférentiellement, on utilisera un agent présentant une activité dans le domaine des antirides, tel qu'un agent anti-radicalaire ou antioxydant, ou un agent stimulant la synthèse de macromolécules dermiques, ou encore un agent stimulant le métabolisme énergétique. Plus particulièrement, le principe actif est choisi parmi les vitamines, les phytostérols, les flavonoïdes, la DHEA et/ou un de ses précurseurs ou un de ses dérivés chimiques ou biologiques, un inhibiteur de métalloproteinase, ou un rétinoïde. En outre, des additifs tels que des agents épaississants, émulsifiants, humectants, émollients, des parfums, des antioxydants, des agents filmogènes, chélatants, séquestrants, conditionnants....peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids, par rapport au poids total de la composition.

Enfin, l'invention se rapporte à une composition comprenant ledit hydrolysat peptidique afin d'augmenter l'activité du protéasome et d'améliorer la dégradation par le protéasome des protéines endommagées.

Un troisième objet de l'invention se rapporte à l'utilisation d'une composition cosmétique comprenant ledit hydrolysat peptidique enrichi et un milieu cosmétiquement acceptable pour prévenir et/ou traiter les signes cutanés du vieillissement et du photo-vieillissement.

Les « signes cutanés du vieillissement » incluent, mais ne se limitent pas à, toutes les manifestations visibles sur la peau causées par le vieillissement. On entend en particulier les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, on entend aussi par « signes cutanés du vieillissement », les pores élargis, les imperfections, la décoloration, les taches de vieillesse, les kératoses, la perte de collagène, et autres changements du derme et de l'épiderme, mais également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme. Par « photo-vieillissement » on entend le vieillissement prématuré de la peau causé par l'exposition prolongée et cumulative au soleil.

La présente invention se rapporte donc à l'utilisation d'une composition pour traiter ou prévenir les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique.

Un autre objet de l'invention concerne l'utilisation d'une composition selon l'invention pour protéger la peau contre les agressions dues aux rayonnements UV.

Enfin, l'invention se rapporte à l'utilisation d'une composition comprenant l'hydrolysat peptidique pour augmenter l'activité du protéasome et améliorer la dégradation par le protéasome des protéines endommagées.

Un dernier objet de la présente invention se rapporte à un procédé de traitement cosmétique consistant à appliquer topiquement sur la peau ou les phanères à traiter, une composition comprenant une quantité efficace d'hydrolysat peptidique enrichi selon l'invention, pour prévenir et/ou traiter les signes cutanés du vieillissement et du photo-vieillissement. En outre, ce procédé de traitement cosmétique peut être appliqué avant le coucher de façon à nettoyer les cellules et la peau pendant le cycle de renouvellement cellulaire. En effet, durant la nuit, la peau privilégie les fonctions de renouvellement ainsi que les processus métaboliques de synthèse. Par conséquent, l'application de la composition telle que revendiquée, en respectant le rythme biologique de la peau permet d'obtenir un effet rajeunissant, stimulant le renouvellement cellulaire, et régénérant.

Les exemples suivants décrivent et démontrent l'efficacité de composés peptidiques tels que décrits selon l'invention mais ne doivent pas être interprétés comme une limitation de la présente invention.

### Exemple 1 : Préparation d'un hydrolysat peptidique à partir de tourteaux de maïs (Zea mays L.)

Le tourteau de maïs (*Zea mays L*.) est mis en solution dans 10 volumes d'eau en présence de 2 % de POLYCLAR® 10 (polyvinylpolypyrrolidone - PVPP - insoluble). Le mélange est ajusté à un pH compris entre 6 et 8 avec une solution aqueuse de soude 1 M. Après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de maïs est récupérée. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification de l'hydrolysat brut commence par des filtrations successives à l'aide de filtres-plaques Seitz-Orion de porosité décroissante (jusqu'à 0.2 µm) afin d'obtenir une solution jaune, brillante et limpide, qualifiée d'hydrolysat 1.

A cette étape, l'hydrolysat 1 de maïs est caractérisé par un extrait sec titrant de 20 à 30 g/kg, un taux de protéines de 20 à 25 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de maïs est chauffé à 70°C pendant 10 minutes dans des conditions réductrices dénaturantes dans un tampon de préparation d'échantillon NuPAGE® LDS. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe des protéines de poids moléculaire inférieur à 6 kDa.

L'hydrolysat 1 est ensuite purifié en éliminant les protéines de haut poids moléculaire par ultrafiltration à l'aide de la cassette Pellicon® 2 Biomax 5 kDa afin de ne conserver que les composés de nature peptidique inférieurs à 5 kDa.

Après cette purification finale, on procède à une phase de dilution afin d'obtenir un hydrolysat peptidique caractérisé par un taux de protéines compris entre 3,5 et 5,5 g/l Cet hydrolysat peptidique enrichi en peptides bioactifs correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa dans les conditions suivantes :
- Gradient Méthanol
- Colonne Uptisphere OPB 125 x 3 mm
- Solvant A: eau grade HPLC contenant 0.1 % d'acide heptafluorobutyrique (HFBA)
- Solvant B: méthanol grade HPLC contenant 0.1% d'acide heptafluorobutyrique (HFBA)
- Gradient: 100% à 15% solvant A en 35 min

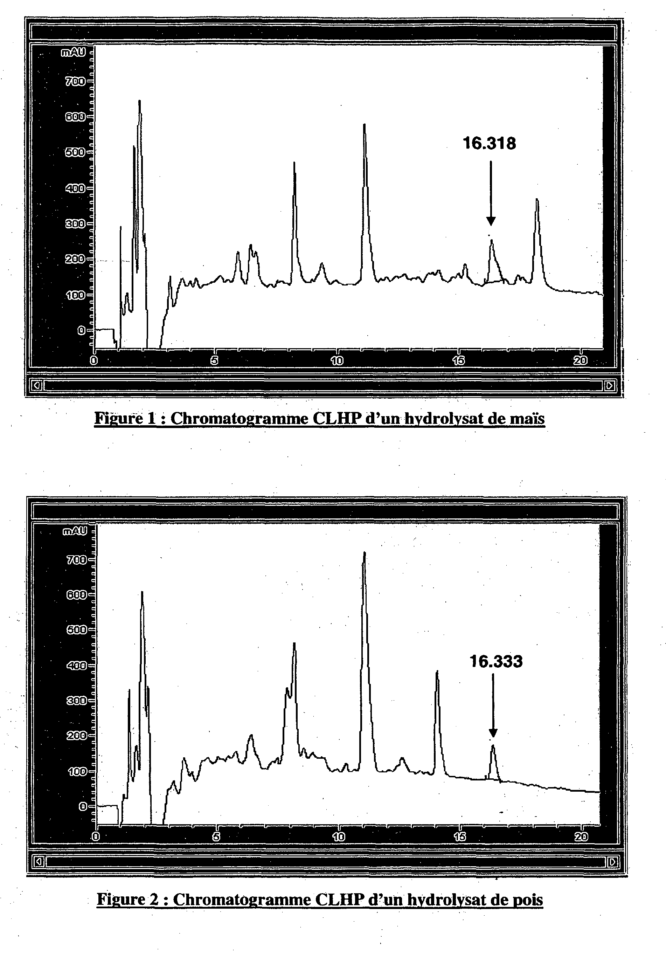

Un exemple de chromatogramme obtenu par CLHP (chromatographie en phase liquide à haute pression), avec mise en évidence du pic correspondant aux peptides bioactifs est donné à la figure 1.

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique du peptide bioactif.

### Exemple 2 : Préparation d'un hydrolysat peptidiques enrichi en peptides bioactifs à partir de pois (Pisum sativum L.)

L'hydrolysat peptidique est obtenu à partir d'un extrait de plantes de l'espèce *Pisum sativum L*. Bien entendu l'extrait peut être préparé à partir de plantes d'au moins l'une quelconque des nombreuses variétés et espèces appartenant au genre Pisum.

Dans une première étape, 1 kg de pois décortiqués sont délipidés par l'action d'un solvant organique : l'hexane.

La farine de pois ainsi obtenue est mise en solution dans 10 volumes d'eau en présence de 2 % de POLYCLAR® 10 (polyvinylpolypyrrolidone - PVPP - insoluble). Le mélange est ajusté à un pH compris entre 6 et 7 avec une solution aqueuse de soude 1 M.

Après ajustement du pH, il est rajouté 2 % de flavourzym® dans le milieu réactionnel. L'hydrolyse est obtenue après 2 heures d'agitation à 50°C. On procède à l'inactivation de l'enzyme en chauffant la solution à 80°C pendant 2 heures. Le mélange réactionnel ainsi obtenu correspond à l'extrait de pois. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'hydrolysat de pois est caractérisé par un sec titrant de 70-80 g/kg, un taux de protéines de 55-65 g/l, un taux de sucres de 2-5 g/l et un taux de polyphénol de 1-3 g/l.

La nature protéique de cet hydrolysat est mise en évidence par électrophorèse sur gel de polyacrylamide. Pour cette analyse, on utilise les gels NuPAGE® Bis-Tris Pre-cast (Invitrogen). L'hydrolysat peptidique de pois est chauffé à 70°C pendant 10 minutes dans des conditions réductrices dénaturantes dans un tampon de préparation d'échantillon NuPAGE® LDS. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 2 grandes familles de protéines : la 1ère famille correspond à des protéines de poids moléculaire de 20 à 25 kDa et la dernière famille à des protéines de poids moléculaires inférieurs à 5kDa.

Cette solution est alors purifiée en éliminant les protéines de poids moléculaires supérieurs à 5 kDa à l'aide d'une filtration à flux tangentiel. Pour cela, l'hydrolysat de pois est pompé sous pression à travers un support Pellicon® équipé de cassette Pellicôn® 2 Biomax 30 kDa. Ce 1er filtrat est récupéré pour être ensuite filtré à travers une autre cassette Pellicon® 2 Biomax 5 kDa. En fin de purification, on obtient un hydrolysat peptidique de pois jaune-beige, brillant et limpide. Il est caractérisé par un sec de 50 à 55 g/kg, une teneur en protéines de 50 à 52 g/l.

Cette solution est alors analysée en chromatographie liquide haute pression à l'aide d'un appareil HP1100 piloté par le logiciel ChemStation. La colonne utilisée lors de l'élution de l'extrait de pois est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont pu être isolées.

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs. Un exemple de chromatogramme obtenu par CLHP (chromatographie en phase liquide à haute pression), avec mise en évidence du pic correspondant aux peptides bioactifs est donné à la figure 2.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 3 : Préparatio. d'un hydrolysat peptidique enrichi en peptides bioactifs à partir de levures Saccharomyces cerevisiae

L'hydrolysat peptidique peut être obtenu à partir d'un extrait de levures de l'espèce *Saccharomyces cerevisiae.* Les levures sont cultivées dans un milieu adapté à leur développement, de préférence en présence de lactose, puis centrifugées pour récupérer une biomasse. La biomasse de saccharomyces est mise en solution dans 10 volumes d'eau en présence de 2. % de POLYCLAR® 10 (polyvinylpyrrolidone - insoluble) et 0,2 % de charbon actif. Le mélange est ajusté à un pH compris entre 6 et 7,5 avec une solution aqueuse de soude 1 M.

Après ajustement du pH, il est rajouté 2 % de papaïne dans le milieu réactionnel. L'hydrolyse est obtenue après 2 heures d'agitation à 55°C. On procède à l'inactivation de l'enzyme en chauffant la solution à 80°C pendant 2 heures. Après centrifugation, le mélange réactionnel correspondant à l'extrait de saccharomyces est alors obtenu. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'extrait de saccharomyces est caractérisé par un sec de 25 à 35 g/kg, un taux de protéines de 10 à 15 g/l et un taux de sucres de 5-10 g/l.

La nature protéique de cet extrait est mise en évidence par électrophorèse sur gel de polyacrylamide. Pour cette analyse, les gels NuPAGE® Bis-Tris Pre-cast (Invitrogen) sont utilisés. L'hydrolysat peptidique est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 3 grandes familles de protéines : la 1ère famille correspond à des protéines de poids moléculaire supérieur à 75 kDa, la 2ème famille à des protéines de 20 à 25 kDa et la dernière famille à des protéines de poids moléculaire inférieur à 5kDa.

Cette solution est alors purifiée en éliminant les protéines de poids moléculaire supérieur à 5 kDa à l'aide de filtration à flux tangentiel. Pour cela, l'hydrolysat peptidique de saccharomyces est pompé sous pression à travers un support Pellicon® équipé de cassette Pellicon® 2 Biomax 50 kDa. Ce 1er filtrat est récupéré pour être ensuite filtré à travers une seconde cassette Pellicon® 2 Biomax 10 kDa. Il est alors récupéré un second filtrat qui est encore élué à travers une dernière cassette Pellicori® 2 Biomax 5 kDa. En fin de purification, on obtient un extrait végétal de saccharomyces beige, brillant et limpide. Il est caractérisé par un sec de 35 à 45 g/kg, une teneur en protéines de 30 à 40 g/l.

Cette solution est alors analysée en chromatographie liquide haute pression à l'aide d'un appareil HP1100 piloté par le logiciel ChemStation. La colonne utilisée lors de l'élution de l'hydrolysat de saccharomyces est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont été isolées.

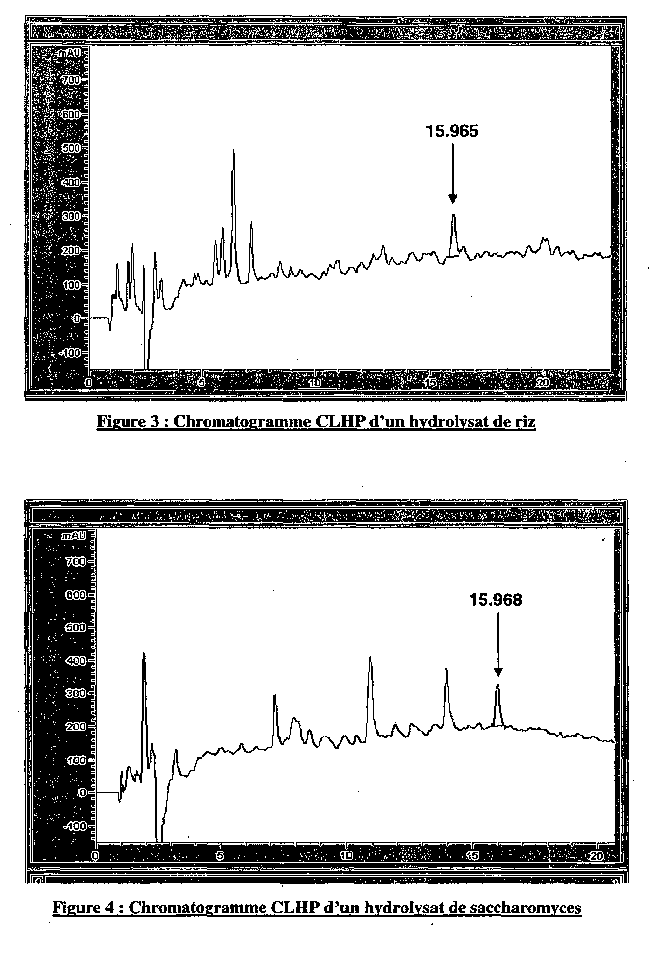

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs. Un exemple de chromatogramme obtenu par CLHP (chromatographie en phase liquide à haute pression), avec mise en évidence du pic correspondant aux peptides bioactifs, est donné à la figure 4.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 4 : Mise en évidence de l'effet activateur de l'hydrolysat peptidique de maïs selon l'exemple 1, sur l'activité enzymatique du proteasome 20S sur des kératinocytes vieillis maintenus en culture

Des kératinocytes vieillis expérimentalement maintenus en culture pendant 15 jours, sont traités avec 1% de notre hydrolysat peptidique issu du maïs réalisé selon l'exemple 1. On étudie alors les activités enzymatiques du protéasome 20S. En effet le protéasome 20S est la sous-unité responsable de l'hydrolyse enzymatique. Trois activités enzymatiques peuvent être étudiées : l'activité trypsine-like, chimotrypsine-like et peptidylglutamyl-peptide hydrolase (PGPH). Nous nous proposons d'étudier ces activités par un dosage enzymatique spécifique de chaque activité.

### Protocole

Les kératinocytes vieillis sont maintenus en culture pendant 15 jours. Le traitement des cellules est effectué par ajout d'une solution à 1 % de notre hydrolysat peptidique directement dans le milieu, renouvelé 3 fois par semaine pendant le temps de l'expérience.

Le dosage de chaque activité a été mis en place en utilisant des substrats spécifiques marqués par un composé fluorescent : le 7-amido-4-méthylcoumarine (AMC). Après clivage, l'AMC dont la longueur d'onde d'excitation est de 350nm, la lecture de la fluorescence est effectuée à 440 nm. L'intensité de la fluorescence est proportionnelle à la quantité de fluorochrome obtenue et en conséquence, cette quantité est proportionnelle à la quantité de substrat hydrolysé.

Le peptide synthétique Boc-Leu-Arg-Arg-AMC est spécifique de l'activité trypsique.

Le peptide synthétique Suc-Leu-Leu-Val-Try-AMC est spécifique de l'activité chymotrypsique.

Le peptide synthétique Z-Leu-Leu-Glu-AMC est spécifique de l'activité peptidylglutamyl-peptide hydrolase.

Ces peptides ont été fournis et marqués par SIGMA ALDRICH, Saint-Louis, MI, USA.

Les cellules sont détachées du support dans un tampon d'extraction. Par la suite, elles sont soniquées pendant 1 minute à 4°C, puis centrifugées à 15000 g pendant 30 minutes à 4°C. Le dosage des protéines est réalisé par le kit BCA (Pierce). Après incubation du lysat cellulaire avec le substrat synthétique spécifique de l'activité étudiée, la fluorescence est lue au spectrophotomètre Synergy (BIOTEK, Vermont, USA) à 440 nm.

### Résultats

Nous constatons que pour les 3 activités étudiées, l'hydrolysat peptidique selon l'exemple 1, a permis d'augmenter l'activité enzymatique du protéasome 20S. L'activité trypsine-like est augmentée de 155.3 % lors du traitement par l'actif, l'activité chymotrypsin-like est augmentée de 130 % et on enregistre une augmentation de 144.6 % pour l'activité peptidylglutamyl-peptide hydrolase.

### Conclusions

L'hydrolysat peptidique enrichi en peptides bioactifs selon l'exemple 1, utilisé à 1 % sur des kératinocytes vieillis expérimentalement en culture permet d'augmenter les activités enzymatiques spécifiques du protéasomes 20S.

L'expérience a été réalisée plusieurs fois, et un test statistique (test statistique t-Student) a pu être réalisé. L'augmentation des activités est significative pour l'étude de l'activité trypsine-like, chymotrypsin-like (p=0.033 et p=0.0477 respectivement), et hautement significative pour l'activité peptidylglutamyl-peptide hydrolase (p=0.00053).

### Exemple 5 : Mise en évidence de l'effet antivieillissement de l'hydrolysat peptidiques de levure selon l'exemple 3 sur des kératinocytes vieillis en culture

Une étude de l'effet antivieillissement de l'hydrolysat peptidique de levure a été réalisée en évaluant l'expression de la protéine bêta-galactosidase sur des kératinocytes vieillis expérimentalement en culture. En effet, l'activité bêta-galactosidase est connue pour être présente dans les cellules sénescentes alors qu'on ne trouve pas d'activité galactosidase dans les cellules pré-sénescentes, quiescentes ou immortelles.

### Protocole

Des kératinocytes vieillis expérimentalement dans des labteck 8 puits sont mis en culture et sont maintenus pendant 20 jours en présence ou non de 1 % d'hydrolysat peptidique de levure enrichi en peptides bioactifs. Le traitement est effectué 3 fois par semaine par ajout direct dans le milieu.

Des cellules non traitées sont maintenues en culture pendant le même temps de l'expérience et serviront de contrôle. Le jour du marquage, les cellules sont rincées, fixées dans un mélange 2 % glutaraldéhyde - 2 % formaldéhyde pendant 3 minutes. Les cellules sont ensuite rincées et on applique 300 µl de 5-bromo-4-chloro-3 indolyl β-D-galactosidase appelé communément X-gal (substrat de la bêta-galactosidase). L'incubation s'effectue pendant 24 heures dans l'incubateur à CO₂, puis les cellules sont rincées et la labteck est rapidement montée dans un milieu adéquat. L'observation s'effectue au microscope à transmission. Le principe est simple : lorsque les cellules sont sénescentes et contiennent la bêta-galactosidase, le substrat X-gal est clivé en un produit bleu insoluble. L'activité bêta-galactosidase est mise en évidence par une coloration des cellules bleues. Plus il y a de cellules bleues, plus le nombre de cellules sénescentes est élevé.

### Résultats / Conclusions :

Nous constatons qu'en présence de l'actif c'est-à-dire de l'hydrolysat peptidiques de levure, l'activité bêta-galactosidase est fortement réduite dans les cellules traitées comparée aux cellules non traitées.

Par conséquent, l'hydrolysat peptidique selon l'invention possède un effet antivieillissement sur des kératinocytes en culture vieillis expérimentalement pendant 20 jours de culture.

### Exemple 6 : Evaluation de la carbonylation des protéines sur des fibroblastes traités par l'hydrolysat peptidique de riz et soumis à des rayonnements ultraviolets (UVB)

### Protocole :

Des fibroblastes humains normaux en culture sont ensemencés dans des boites de diamètres 100. Lorsque les cellules ont atteint 70 % de confluence, les cellules sont traitées 48 heures avec un hydrolysat peptidique de riz enrichi en peptides bioactifs dilué à 1 % dans le milieu. Les cellules sont soumises à l'irradiation UVB à 100mj/cm², puis remises 48 heures supplémentaires en présence du principe actif. Des boîtes contrôle avec des cellules non traitées par le principe actif mais irradiées servent de témoin. Les cellules sont rincées puis détachées du support à l'aide d'un tampon d'extraction adéquat. Les protéines ainsi extraites, sont centrifugées à 4°C à 10000 rpm pendant 10 minutes avant d'être dosées par le kit de dosage des protéines BCA (Pierce). La carbonylation des protéines est réalisée par un test basé sur l'immunodétection des groupements carbonylés préalablement dérivés par le 2,4-dinitrophénylhydrazine (DNP) (SIGMA).

Selon la réaction :
Protéine-C=O + H₂N-NH-₂₋₄DNP → Protéine-C=N-NH_{2,4}-DNP + H₂O

Brièvement, 15 µl de l'échantillon sont mis à réagir avec 45 µl de DNP pendant 45 minutes à température ambiante. Ensuite, 5 µl du mélange est dilué dans 1 ml de phosphate buffer saline et 200 µl de cette dilution sont mis dans une plaque 96 puits over night à 4°C en présence de 150 µl de BSA (fraction V).

Après 3 lavages en tampon phosphate saline (PBS), l'anticorps biotinylé de lapin anti-dinitrophenyl (CALBIOCHEM) est diluée au 1/5000^{ème} dans du tampon 0.1 % sérum albumine en présence de 0.1 % Tween 20 et incubé dans les microplaques 1 heure à 37°C. Après 3 lavages, on incube le complexe streptavidine-péroxydase (DAKO) dilué au 1/3000^{ème} dans du tampon 0.1 % sérum albumine en présence de 0.1 % Tween 20 et incubé dans les microplaques pendant 1 heure à température ambiante. Après 3 lavages, la révélation est effectuée grâce à 200 µl de tétramethylbenzidine (TMB, SIGMA) 25 minutes à température ambiante. Ensuite, l'ajout de 100 µl d'acide sulfurique à 2,5 M permet de stopper la réaction. La lecture de la DO s'effectue à 490 nm. Afin de convertir la DO obtenue en groupements carbonylés présents dans les échantillons, une courbe étalon a été établie en faisant varier des proportions de 0 à 100 % de BSA oxydée.

### Résultats :

En présence d'UVB les cellules non traitées sont fortement carbonylées et augmentent de 110 % par rapport aux cellules non irradiées et non traitées. En présence de l'hydrolysat peptidique de riz enrichi en peptides bioactifs à 1 %, le taux de carbonylation est diminué de 34 %. L'expérience a été réalisée plusieurs fois et un test statistique (test statistique t Student) a pu être réalisé. La diminution de la carbonylation est significative et p= 0.0298.

En conclusion, le principe actif selon l'invention permet de protéger les cellules contre les effets néfastes des UV c'est-à-dire contre leurs effets oxydatifs. L'hydrolysat peptidique de riz permet de diminuer de plus de 34 % l'oxydation des protéines.

### Exemple 7 : Test clinique

### Protocole pour l'évaluation clinique

12 volontaires âgés de 29 à 56 ans ont appliqué soit un placebo, soit l'hydrolysat peptidique de levures enrichi en peptides bioactifs selon l'exemple 3, 2 fois par jour matin et soir, à une dose de 2 mg/cm² pendant 24 jours. Une évaluation clinique des résultats a permis de mesurer plusieurs paramètres des rides et ridules.

La mesure des rides et ridules a été réalisée grâce au QUANTIRIDE qui est une méthode d'évaluation qui permet de mesurer le nombre, la longueur et la profondeur des rides en effectuant une réplique de la peau avant et après traitement à l'aide d'un polymère silicone.

Les résultats sont compilés dans les tableaux ci-dessous :

### Résultats quantification ride

| **Longueur des les rides** | **Temps** | **Mesure (mm)** | **Wilcoxon** | **% de volontaires avant une amélioration** |
|---|---|---|---|---|
| **Hydrolysat** | J 23-J 0 | -0.104 | 0.017 | 83.3% |
| **Placebo** | J23-J 0 | 0,0029 | N/A | N/A |
| | | | | |

| **Nombre de rides** | **Temps** | **Mesure (mm)** | **Wilcoxon** | **% de volontaires avant une amélioration** |
|---|---|---|---|---|
| **Hydrolysat** | J 23-J 0 | -9.5833 | 0.0249* | 75% |
| **Placebo** | J 23-J 0 | 5.8333 | N/A | N/A |
| | | | | |

| **Profondeur des rides** | **Temps** | **Mesure (mm)** | **Wilcoxon** | **% de volontaires avant une amélioration** |
|---|---|---|---|---|
| **Hydrolysat** | J 23-J 0 | -6,5557 | 0,0075 | 75% |
| **Placebo** | J 23-J 0 | 3,6284 | N/A | N/A |

### Conclusions:

Après 24 jours de traitement, nous observons une diminution statistique de la longueur totale des rides chez 83.3 % des sujets traités, ainsi qu'une diminution du nombre de rides chez 75 % des sujets traités. Concernant la longueur des rides, on constate une différence significative entre l'hydrolysat peptidique de levures enrichi en peptides bioactifs et le placebo (p=0,017). Pour la profondeur des rides, la différence entre le principe actif et le placebo est aussi significative (p=0,0075) et est observée chez 75 % des volontaires.

## Revendications

1. Hydrolysat peptidique activateur du protéasome, **caractérisé en ce que** l'hydrolysat est issu de l'hydrolyse de levures du genre *Saccharomyces*, et plus particulièrement de l'espèce *Saccharomyces cerevisiae*, ou de pois (*Pisum sativum*) et est enrichi en peptides bioactifs de poids moléculaire inférieur à 6 kDa, comportant de 3 à 5 acides aminés, chaque peptide bioactif comprenant au moins un résidu acide aspartique, un résidu cystéine et un résidu arginine.

2. Hydrolysat peptidique enrichi selon la revendication 1, **caractérisé en ce que** le peptide bioactif est de formule générale (I) :
X₁ - [Asp, Cys, Arg] - X₂
dans laquelle,
X₁ est une asparagine, une lysine, un aspartate, une valine, une arginine, ou est absent,
X₂ est une histidine, une lysine, une arginine, ou est absent.

3. Hydrolysat peptidique enrichi selon la revendication 2, **caractérisé en ce que** le peptide bioactif correspond à une des formules suivantes :
(SEQ ID n°1) Arg - Asp - Cys - Arg - Arg
(SEQ ID n°2) Asn - Asp - Cys - Arg - Lys
(SEQ ID n°3) Asp - Cys - Arg - His
(SEQ ID n°4) Val - Asp - Cys - Arg
(SEQ ID n°5) Asp - Cys - Arg.

4. Hydrolysat peptidique enrichi selon l'une quelconque des revendications précédentes, **caractérisé en ce qu'**il contient entre 0,5 et 5,5 g/l de composés de nature peptidique.

5. Hydrolysat peptidique enrichi selon l'une quelconque des revendications précédentes, **caractérisé en ce qu'**il est utilisé à titre de médicament.

6. Composition cosmétique comprenant à titre de principe actif ledit hydrolysat peptidique enrichi selon l'une des revendications 1 à 4.

7. Composition selon la revendication 6, **caractérisée en ce qu'**elle se présente sous une forme adaptée à l'application par voie topique et comprend un milieu cosmétiquement acceptable.

8. Composition selon l'une des revendications 6 ou 7, **caractérisée en ce que** ledit hydrolysat peptidique enrichi est présent dans la composition en une quantité représentant de 0,0001 % à 20 % du poids total de la composition, et préférentiellement en une quantité représentant de 0,05 % à 5 % du poids total de la composition.

9. Composition selon l'une quelconque des revendications 6 à 8, **caractérisée en ce qu'**elle contient en outre, au moins un autre principe actif favorisant l'action dudit hydrolysat peptidique enrichi choisi parmi les vitamines, les phytostérols, les flavonoïdes, la DHEA, un inhibiteur de métalloprotéinase, un rétinoïde.

10. Utilisation cosmétique d'une composition telle que définie dans l'une des revendications 6 à 9, pour augmenter l'activité du protéasome et améliorer la dégradation par le protéasome des protéines endommagées.

11. Utilisation cosmétique d'une composition telle que définie dans l'une des revendications 6 à 9, pour prévenir et/ou traiter les signes cutanés du vieillissement et du photo-vieillissement tels que les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique.

12. Utilisation cosmétique selon la revendication 11, **caractérisée en ce que** ladite composition permet de protéger la peau contre les agressions dues aux rayonnements UV.

13. Procédé de traitement cosmétique **caractérisé en ce que** l'on applique topiquement sur la peau ou les phanères à traiter, une composition comprenant une quantité efficace d'hydrolysat peptidique enrichi tel que défini dans l'une des revendications 1 à 4, pour prévenir et/ou traiter les signes cutanés du vieillissement et du photo-vieillissement.

## Patentansprüche

1. Proteasomaktivierendes Peptidhydrolysat, **dadurch gekennzeichnet, dass** das Hydrolysat aus der Hydrolyse von Hefen der Gattung *Saccharomyces* und insbesondere der Spezies *Saccharomyces cerevisiae* oder von Erbsen (*Pisum sativum*) hervorgeht und mit bioaktiven Peptiden mit einem Molekulargewicht von weniger als 6 kDa, die 3 bis 5 Aminosäuren umfassen, angereichert ist, wobei jedes bioaktive Peptid mindestens einen Asparaginsäurerest, einen Cysteinrest und einen Argininrest umfasst.

2. Angereichertes Peptidhydrolysat nach Anspruch 1, **dadurch gekennzeichnet, dass** das bioaktive Peptid die allgemeine Formel (I) aufweist:
X₁- [Asp, Cys, Arg]-X₂,
in der
X₁ ein Asparagin, ein Lysin, ein Aspartat, ein Valin, ein Arginin oder abwesend ist,
X₂ ein Histidin, ein Lysin, ein Arginin oder abwesend ist.

3. Angereichertes Peptidhydrolysat nach Anspruch 2, **dadurch gekennzeichnet, dass** das bioaktive Peptid einer der folgenden Formeln entspricht:
(SEQ ID Nr. 1) Arg-Asp-Cys-Arg-Arg
(SEQ ID Nr. 2) Asn-Asp-Cys-Arg-Lys
(SEQ ID Nr. 3) Asp-Cys-Arg-His
(SEQ ID Nr. 4) Val-Asp-Cys-Arg
(SEQ ID Nr. 5) Asp-Cys-Arg.

4. Angereichertes Peptidhydrolysat nach einem der vorhergehenden Ansprüche, **dadurch gekennzeichnet, dass** es zwischen 0,5 und 5,5 g/l natürlicher Peptidverbindungen enthält.

5. Angereichertes Peptidhydrolysat nach einem der vorhergehenden Ansprüche, **dadurch gekennzeichnet, dass** es als Arzneimittel verwendet wird.

6. Kosmetische Zusammensetzung, die das besagte angereicherte Peptidhydrolysat nach einem der Ansprüche 1 bis 4 als Wirkstoff umfasst.

7. Zusammensetzung nach Anspruch 6, **dadurch gekennzeichnet, dass** sie in einer zum topischen Auftrag geeigneten Form dargereicht wird und ein kosmetisch akzeptables Medium umfasst.

8. Zusammensetzung nach einem der Ansprüche 6 oder 7, **dadurch gekennzeichnet, dass** das besagte Peptidhydrolysat in der Zusammensetzung in einer Menge vorliegt, die 0,0001 % bis 20 % des Gesamtgewichts der Zusammensetzung ausmacht, und vorzugsweise in einer Menge vorliegt, die 0,05 % bis 5 % des Gesamtgewichts der Zusammensetzung ausmacht.

9. Zusammensetzung nach einem der Ansprüche 6 bis 8, **dadurch gekennzeichnet, dass** sie außerdem mindestens einen anderen Wirkstoff umfasst, der die Wirkung des besagten angereicherten Peptidhydrolysats begünstigt und aus Vitaminen, Phytosterolen, Flavonoiden, DHEA, einem Metalloproteinasehemmer, einem Retinoid ausgewählt ist.

10. Kosmetische Verwendung einer wie in einem der Ansprüche 6 bis 9 definierten Zusammensetzung zum Verstärken der Aktivität des Proteasoms und Verbessern des Abbaus von beschädigten Proteinen durch das Proteasom.

11. Kosmetische Verwendung einer wie in einem der Ansprüche 6 bis 9 definierten Zusammensetzung zum Verhindern und/oder Behandeln von Zeichen der Alterung und Lichtalterung der Haut, wie Falten, tiefen und groben Falten, Fältchen, Schrunden, der Erschlaffung von Haut- und Unterhautgeweben, dem Verlust der Hautelastizität und Atonie, dem Verlust der Festigkeit und der Tonizität und Hautatrophie.

12. Kosmetische Verwendung nach Anspruch 11, **dadurch gekennzeichnet, dass** die besagte Zusammensetzung das Schützen der Haut vor Angriffen aufgrund von UV-Strahlen ermöglicht.

13. Verfahren zur kosmetischen Behandlung, **dadurch gekennzeichnet, dass** eine Zusammensetzung, die eine wirksame Menge eines wie in dem der Ansprüche 1 bis 4 definierten angereicherten Peptidhydrolysats umfasst, zum Verhindern und/oder Behandeln von Zeichen der Alterung und Lichtalterung der Haut topisch auf die zu behandelnde Haut oder die zu behandelnden Hautanhangsgebilde aufgebracht wird.

## Claims

1. Proteasome-activating peptide hydrolysate, **characterised in that** the hydrolysate is obtained from the hydrolysis of yeasts of the *Saccharomyces* genus, and more particularly the species *Saccharomyces cerevisiae,* or pea (*Pisum sativum*) and enriched with bioactive peptides having a molecular weight less than 6 kDa, comprising 3 to 5 amino acids, each bioactive peptide comprising at least one aspartic acid residue, one cysteine residue and one arginine residue.

2. Enriched peptide hydrolysate according to claim 1, **characterised in that** the bioactive peptide has the following general formula (I):
X₁ - [Asp, Cys, Arg] - X₂
wherein,
X₁ is an asparagine, a lysine, an aspartate, a valine, an arginine, or is absent,
X₂ is a histidine, a lysine, an arginine, or is absent.

3. Enriched peptide hydrolysate according to claim 2, **characterised in that** the bioactive peptide corresponds to one of the following formulas:
(SEQ ID No. 1) Arg - Asp - Cys - Arg - Arg
(SEQ ID No. 2) Asn - Asp - Cys - Arg - Lys
(SEQ ID No. 3) Asp - Cys - Arg - His
(SEQ ID No. 4) Val - Asp - Cys - Arg
(SEQ ID No. 5) Asp - Cys - Arg.

4. Enriched peptide hydrolysate according to any of the above claims, **characterised in that** it contains between 0.5 and 5.5 g/l of peptide type compounds.

5. Enriched peptide hydrolysate according to any of the above claims, **characterised in that** it is used as a medicinal product.

6. Cosmetic composition comprising as an active substance said enriched peptide hydrolysate according to any of claims 1 to 4.

7. Composition according to claim 6, **characterised in that** it is presented in a form suitable for topical application and comprises a cosmetically acceptable medium.

8. Composition according to any of claims 6 or 7, **characterised in that** said enriched peptide hydrolysate is present in the composition in a quantity representing from 0.0001% to 20% of the total weight of the composition, and preferentially in a quantity representing from 0.05% to 5% of the total weight of the composition.

9. Composition according to any of claims 6 to 8, **characterised in that** it further contains at least one further active substance promoting the action of said enriched peptide hydrolysate chosen from vitamins, plant sterols, flavonoids, DHEA, a metalloproteinase inhibitor, a retinoid.

10. Cosmetic use of a composition as defined in any of claims 6 to 9, for increasing proteasome activity and enhancing degradation of damaged proteins by the proteasome.

11. Cosmetic use of a composition as defined in any of claims 6 to 9, for preventing and/or treating the skin signs of ageing and photo-ageing such as wrinkles, deep and coarse wrinkles, lines, cracks, slackening of cutaneous and subcutaneous tissue, loss of skin elasticity and atony, loss of firmness and tone, and dermal atrophy.

12. Cosmetic use according to claim 11, **characterised in that** said composition is suitable for protecting the skin against damage caused by UV rays.

13. Cosmetic treatment method **characterised in that** a composition comprising an effective quantity of enriched peptide hydrolysate as defined in any of claims 1 to 4 is applied topically onto the skin or skin appendages to be treated, for preventing and/or treating the skin signs of ageing and photo-ageing.


### SEQUENCE LISTING

<110> ISP Investments INC.
<120> HYDROLYSATS PEPTIDIQUES ACTIVATEURS DU PROTEASOME ET COMPOSITIONS LES CONTENANT
<130> Bv PCT 09-126
<150> FR 09 01978
   <151> 2009-04-23
<160> 5
<170> PatentIn version 3.3
<210> 1
   <211> 5
   <212> PRT
   <213> Plant OR saccharomyces cerevisiae
<400> 1
   Arg Asp Cys Arg Arg
   1 5
<210> 2
   <211> 5
   <212> PRT
   <213> Plant OR saccharomyces cerevisiae
<400> 2
   Asn Asp Cys Arg Lys
   1 5
<210> 3
   <211> 4
   <212> PRT
   <213> Plant OR saccharomyces cerevisiae
<400> 3
   Asp Cys Arg His
   1
<210> 4
   <211> 4
   <212> PRT
   <213> Plant OR saccharomyces cerevisiae
<400> 4
   Val Asp Cys Arg
   1
<210> 5
   <211> 3
   <212> PRT
   <213> Plant OR Saccharomyces cerevisiae
<400> 5
   Asp Cys Arg